(19) Europäisches Patentamt
European Patent Office
Office européen des brevets

(11) Veröffentlichungsnummer: **0 303 920 B1**

(12) # EUROPÄISCHE PATENTSCHRIFT

(45) Veröffentlichungstag der Patentschrift: **18.03.92**

(51) Int. Cl.$^5$: **C07D 307/86**, C07D 405/06, C07D 405/12, A61K 31/34, A61K 31/40, A61K 31/44

(21) Anmeldenummer: **88112833.4**

(22) Anmeldetag: **06.08.88**

(54) **Neue Benzofuranderivate und diese enthaltende therapeutische Mittel.**

(30) Priorität: **20.08.87 DE 3727736**

(43) Veröffentlichungstag der Anmeldung:
**22.02.89 Patentblatt 89/08**

(45) Bekanntmachung des Hinweises auf die Patenterteilung:
**18.03.92 Patentblatt 92/12**

(84) Benannte Vertragsstaaten:
**BE CH DE FR GB IT LI NL SE**

(56) Entgegenhaltungen:
**DE-A- 2 238 115**
**DE-A- 2 730 593**
**DE-B- 2 235 941**
**FR-A- 2 387 227**
**DE-A- 2 405 278**
**DE-B- 1 933 178**
**FR-A- 2 387 226**
**FR-A- 2 447 378**

(73) Patentinhaber: **BASF Aktiengesellschaft**
**Carl-Bosch-Strasse 38**
**W-6700 Ludwigshafen(DE)**

(72) Erfinder: **Schlecker, Rainer, Dr.**
**Suedring 8**
**W-6719 Bissersheim(DE)**
Erfinder: **Raschack, Manfred, Dr.**
**Donnersbergstrasse 7**
**W-6714 Weisenheim(DE)**
Erfinder: **Gries, Josef, Dr.**
**Roemerweg 43**
**W-6706 Wachenheim(DE)**

EP 0 303 920 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Benzofuranderivate der Formeln I und Ia und diese enthaltende therapeutische Mittel zur Behandlung von Herz-Kreislauf-Erkrankungen,koronaren Herzerkrankungen, Vasospasmen und Hypertonie.

In den DOS 2 235 941 und 2 238 115 sind Benzofuranderivate beschrieben, die eine starke calciumantagonistische Aktivität in vitro aufweisen, enteral jedoch nur schwach wirksam sind.

Es wurde nun überraschend gefunden, daß die Benzofuranderivate der Formeln I und Ia

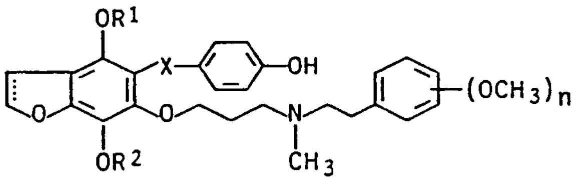

I

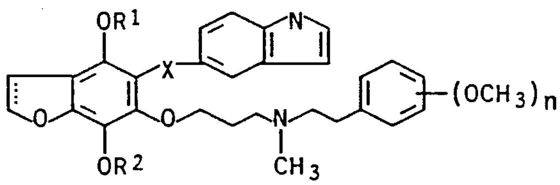

Ia

worin $R^1$ und $R^2$ unabhängig voneinander Wasserstoff, Alkyl oder Phenylalkyl mit jeweils 1 bis 4 C-Atomen im Alkylrest bedeuten,

x eine der Gruppen $-CO-CH=CH-$, $-CO-CH_2-CH_2-$ oder $-CHOH-CH_2-CH_2-$ darstellt,

n die Zahl 1 oder 2 ist und

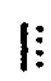 eine Einfach- oder Doppelbindung

darstellt,

bei Erhalt der calciumantagonistischen Aktivität eine deutlich höhere orale Verfügbarkeit besitzen.

Die erfindungsgemäßen Verbindungen der Formeln I und Ia können im Prinzip nach Verfahren hergestellt werden, wie sie in der DE-A-3 710 469 beschrieben sind.

Die Verbindungen der allgemeinen Formeln I und Ia, in der X für die Gruppe $-CO-CH_2CH_2-$ steht, können hergestellt werden aus den Verbindungen der Formeln I und Ia, in denen X die Gruppe $-CO-CH=CH-$ bedeutet, durch katalytische Hydrierung der Doppelbindung nach literaturbekannten Methoden, wie sie beispielsweise in R.N. Rylander, "Catalytic Hydrogenation over Pt-Metals", Acad. Press, New York, S. 282, 1967, beschrieben sind. Als Katalysatoren eignen sich insbesondere Metallkatalysatoren wie Palladium auf Kohle oder Raney-Nickel in Alkohol.

Die Verbindungen der allgemeinen Formeln I und Ia, in denen X für die Gruppe $-CH(OH)-CH_2CH_2-$ steht, können hergestellt werden durch Reduktion der Verbindungen der allgemeinen Formeln I und Ia, in der X $-CH-CH_2CH_2-$ bedeutet, mit Metallhydriden nach an sich bekannten Verfahren, wie sie in Houben-Weyl, Methoden der org. Chemie, 4. Auflage, G. Thieme Verlag Stuttgart 1984, Band 6/1 b, S. 145, beschrieben sind. Als Metallhydride eignen sich beispielsweise $LiAlH_4$ in Ethern oder $NaBH_4$ in Alkoholen wie Ethanol oder Isopropanol.

weiterhin können diese Verbindungen hergestellt werden aus den Verbindungen der Formeln I und Ia, in der X für $-CO-CH=CH-$ steht, durch katalytische Hydrierung, wie sie in Houben-Weyl, Bd. 6/1b, S. 61 beschrieben ist, oder durch Reduktion mit Metallhydriden, wie sie in Houben-Weyl, 4. Aufl. (1981), Bd. 4/1d, S. 297 und in der DE-OS 22 35 941 beschrieben ist.

Die Synthese der Verbindungen der Formeln I und Ia, in der X $-CO-CH=CH-$ bedeutet, erfolgt aus den

Acetophenonen der Formel III durch Kondensation mit aromatischen Aldehyden nach bekannten Methoden, wie sie beispielsweise in Org. Reactions Bd. 16, S. 1 ff, John Wiley Verlag, New York 1968 beschrieben sind. Als Kondensationsmittel können beispielsweise Alkalihydroxide in wäßrig alkoholischer Lösung dienen.

Die Verbindungen der Formel III, in der $R^1$ und $R_2$ = $CH_3$ ist, können erhalten werden durch Alkylierung des o-Hydroxyacetophenons Khellinon II (JACS 72 (1950), 1613) mit Halogenalkylaminen, wie es in Chimie Therapeutique 4 (1973), 475 und der Deutschen Patentanmeldung P 37 10 469.1 beschrieben ist. Weiterhin kann die Alkylierung von II

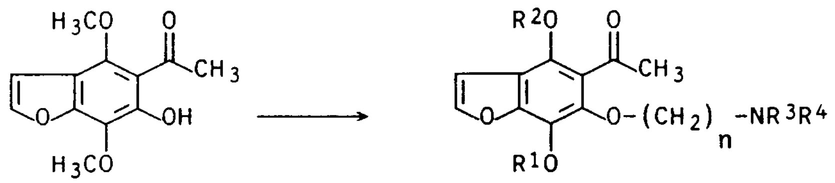

II III

mit Aminoalkoholen unter den Bedingungen der Mitsunobu-Methode (Synth. 1981, 1) erfolgen.

Die Verbindungen der Formel III, in der $R^1$ oder $R^2$ ■$CH_3$ ist, lassen sich herstellen durch Alkylierung der Hydroxyacetophenone IV

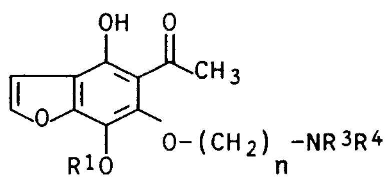

IV

nach den an sich bekannten Methoden der Phenolsynthese (Houben-Weyl Bd. VI/3 S. 49 f). So kann die Alkylierung beispielsweise durchgeführt werden mit Alkylhalogeniden unter Verwendung von Alkalicarbonaten als Base in Aceton als Lösungsmittel oder von Metallhydriden in aprotischen Lösungsmitteln, wie DMF oder THF.

Die Verbindungen IV sind zugänglich durch Hydrogenolyse des Benzylethers V nach bekannten Verfahren (Houben-Weyl Bd. 4/1c, S. 385 f). Die Entbenzylierung wird vorzugsweise bei Raumtemperatur durchgeführt, um eine Hydrierung des Furanrings zu vermeiden.

Die Synthese der Verbindungen V erfolgt aus den Hydroxyacetophenonen VI nach dem für die Herstellung von III ($R^1$ = $R^2$ = $CH_3$ beschriebenen Verfahren.

Das Hydroxyacetophenon VI wird hergestellt durch alkalische Ringspaltung des Pyrons VII nach dem in der DE-A-3 710 469 beschriebenen Verfahren.

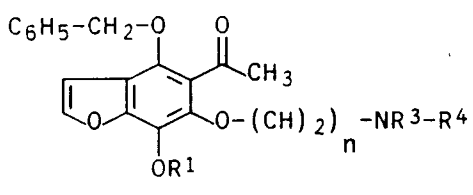

V

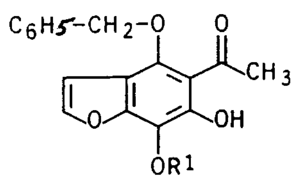

VI

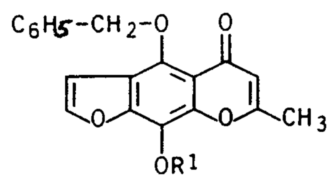

VII

Die Synthese von Verbindungen der allgemeinen Formeln I und Ia, in denen der Furanring hydriert ist, erfolgt in analoger Weise, wobei man ein geeignetes Zwischenprodukt wie beispielsweise II nach an sich bekannten Methoden der katalytischen Hydrierung in das entsprechende Dihydrobenzofuranderivat überführt.

Die Hydrierung erfolgt vorzugsweise bei leicht erhöhter Temperatur von 20 ° C bis 100° C mit Pd oder Pt als Katalysator.

Die erfindungsgemäßen Verbindungen der Formeln I und Ia, in denen X-CHOH-$CH_2$-$CH_2$-bedeutet, besitzen ein Chiralitätszentrum und werden als Racemate erhalten, die durch bekannte Methoden, beispielsweise durch Bildung diastereomerer Salze mit optisch aktiven Hilfsäuren oder Dibenzoylweinsäure,

Campher-10-Sulfonsäure oder Ditolylweinsäure in die optisch aktiven Antipoden getrennt werden können.

Gegebenenfalls werden die erhaltenen erfindungsgemäßen Verbindungen in das Säureadditionssalz einer physiologisch verträglichen Säure überführt. Eine Zusammenstellung üblicher physiologisch verträglicher Säuren kann aus Fortschritte der Arzneimittelforschung 1966, Birkhäuser Verlag, Bd. 10, S. 224 - 285, Deutschland, Schweiz entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether, wie Diethylether, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen der Aminopropanolderivate der Formeln I und Ia durch Auflösen der freien Basen in einer wäßrigen Säurelösung hergestellt werden.

Die erfindungsgemäßen Verbindungen (der allgemeinen Formeln I und Ia) und deren physiologisch verträglichen Säureadditionssalze besitzen wertvolle pharmakologische Eigenschaften. Sie sind hochaktive Ca-Antagonisten und relaxieren dadurch Blutgefäße in vitro. Ferner senken sie bei normotonen und hypertonen Versuchstieren den arteriellen Blutdruck. Außerdem sind sie in der Lage, Herzmuskulatur gegen den bei Sauerstoffmangelbeatmung auftretenden Verlust an energiereichen Phosphaten (z. B. Adenosintriphosphat, ATP) zu schützen, d. h. sie wirken kardioprotektiv.

Aufgrund der ausgeprägten Ca-antagonistischen Wirkung und der überlegenen antihypertensiven bzw. kardioprotektiven Wirkung sind die erfindungsgemäßen Verbindungen geeignet zur enteralen Behandlung von Herz-Kreislauf-Erkrankungen, insbesondere der koronaren Herzerkrankung, von Vasospasmen und Hypertonie.

Die Dosierung hängt vom Alter, Zustand und Gewicht des Patienten sowie von der Applikationsart ab. In der Regel beträgt die tägliche Wirkstoffdosis zwischen etwa 10 und 1000 mg pro Patient und Tag.

Die neuen Verbindungen können in den gebräuchlichen festen galenischen enteralen Applikationsformen angewendet werden, z.B. als Tabletten, Filmtabletten, Kapseln, Pulver, Granulate, Dragees oder Suppositorien. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Zum Nachweis der pharmakologischen Wirkung wurde folgende Methode verwendet:

Antihypertensive Wirkung

Die Substanzen werden männlichen spontan hypertonen Okamoto-Ratten (SHR) (5 bis 10 Tiere/Dosis, Gewicht 270 bis 380 g) oral appliziert. Der systolische Blutdruck wird vor und 2 h nach der Applikation unblutig am Rattenschwanz mit Hilfe von Piezokristallaufnehmern mit einer plethysmographischen Methode ermittelt. Als ED 20 % wird unter Berücksichtigung der Werte unbehandelter Kontrolltiere die Dosis bestimmt, welche den systolischen Blutdruck um 20 % senkt.

Table

| Blood pressure lowering doses (ED 20 %, mg/kg) in conscious spontaneously hypertensive rats (SHR) | | |
|---|---|---|
| Example | Antihypertensive activity SHR p.o. | |
| | ED20 % | R.P.* |
| 1 | 5,0 | 17.2 |
| 2 | 6,7 | 12.7 |
| 3 | 21,5 | 4.0 |
| 5 | 13,1 | 6.5 |
| 6 | 12,6 | 6.8 |
| 8 | 21,5 | 4.0 |
| Piprofurol | 85,5 | 1 |

* Relative potency; Piprofurol = 1.00

Die Ergebnisse belegen, daß die oral applizierten erfindungsgemäßen Substanzen gute hypotensive und antihypertensive Wirkungen besitzen.

Beispiele zur Herstellung der Ausgangsverbindungen

5-Acetyl-4,7-dimethoxy-6-[3-((2-(3,4-dimethoxyphenyl)ethyl) -methyl-amino)propoxy]benzofuran

39 g 5-Acetyl-6-hydroxy-4,7-dimethoxybenzofuran werden mit 40 g 3-Chlorpropyl-(2-(3,4-dimethoxyphenyl)ethyl)methylamin in 250 ml Methylethylketon 14 Stunden unter Rückfluß erhitzt. Die Mischung wird filtriert, das Lösungsmittel abgezogen und der ölige Rückstand in verd. HCl gelöst. Die Wasserphase wird mit Ether gewaschen, mit verd. Natronlauge alkalisch gestellt und mit Ether extrahiert. Nach Abziehen des Lösungsmittels erhält man 45 g rohes öliges Produkt, das ohne Reinigung weiter umgesetzt wird.

4-Benzyloxy-9-methoxy-7-methyl-furo[3, 2-g]chromon

95 g 4-Hydroxy-9-methoxy-7-methyl-furo[3,2-g]chromon werden in 1000 ml Methylethylketon mit 100 g Benzylbromid und 210 g $K_2CO_3$ 15 Stunden unter Rückfluß erhitzt. Die Mischung wird mit 1000 ml $CH_2Cl_2$ versetzt, filtriert und das Lösungsmittel abdestilliert. Der Rückstand liefert nach Behandeln mit Petrolether 123 g VII ($R^1$ = $CH_3$) als gelbliches öl.

5-Acetyl-4-benzyloxy-6-hydroxy-7-methoxy-benzofuran

Zu einer Lösung von 67 g KOH in 1000 ml Wasser werden bei 75°C portionsweise 113 g VII ($R^1$ = $CH_3$) gegeben. Die Mischung wird 3 Stunden unter Rückfluß erhitzt, abgekühlt und filtriert. Das Filtrat wird mit 110 ml konz. Salzsäure angesäuert, der ausgefallene Rückstand abgesaugt und getrocknet. Man erhält 115 g des Zielproduktes.

In analoger Weise wurden hergestellt:

5-Acetyl-4-ethoxy-7-methoxy-6-[3-( (2-(3,4-dimethoxyphenyl)ethyl)-methyl-amino)propoxy]benzofuran, öl
5-Acetyl-4,7-dimethyoxy-6-[3-( (2-(3-methoxyphenyl)ethyl)-methyl-amino)-propoxy]benzofuran, öl
5-Acetyl-4-ethoxy-7-methoxy-6-[3-( (2-(3-methoxyphenyl)ethyl)-methylamino)-propoxy]benzofuran, öl
5-Acetyl-2,3-dihydro-6-hydroxy-4,7-dimethoxybenzofuran
10 g 5-Acetyl-6-hydroxy-4,7-dimethoxybenzofuran wurden in 100 ml Methanol mit 2 g Pd/C (10 %) bei Raumtemperatur und geringem Überdruck hydriert. Nach Beendigung der Wasserstoffaufnahme wurde die Mischung filtriert und das Lösungsmittel abdestilliert. Man erhielt 9,7 g des Zielproduktes. Fp. = 100-101°C.

Beispiele zur Herstellung der erfindungsgemäßen Verbindungen:

Beispiel 1

1-[4, 7-Dimethoxy-6-[3-( (2-(3,4-dimethoxyphenyl)ethyl)-methyl-amino)-prop--oxy]benzofuranyl-5-]-3-(4-hydroxyphenyl)propenon

8 g Acetyl-4,7-dimethoxy-6-[3-((2-(3,4-dimethoxyphenyl)-ethyl)-methyl-amino)propoxy]benzofuran und 4 g 4-Hydroxybenzaldehyd wurden in 60 ml Ethanol/20 g konz. Natronlauge über Nacht bei RT gerührt. Die Mischung wurde auf Eis gegossen, mit verd. Salzsäure neutralisiert und mit $CH_2Cl_2$ extrahiert. Die org. Phase wurde nochmals mit Wasser gewaschen, getrocknet und eingeengt. Der Rückstand wurde mit der äquimolaren Menge Oxalsäure in $CH_2Cl_2$ gelöst. Durch Zugabe von Ether wurde das Produkt als Oxalat ausgefällt.

Ausbeute: 2,8 g, Fp. 60 ° C

In analoger Weise wurden die Beispiele 2 bis 5 in Tabelle 1 synthetisiert. Die Strukturen aller Verbindungen wurden NMR-spektroskopisch gesichert.

Beispiel 6

1-[4-Ethoxy-7-methoxy-6-[3-((2-(3,4-dimethoxyphenyl)ethyl)-methyl-amino)-propoxy]benzofuranyl-5]-3-(4-hydroxyphenyl)propanon

15 g Chalkon aus Beispiel 2 wurden bei RT in 200 ml Methanol mit 1,5 g Pd/C bis zur Aufnahme der berechneten Wasserstoffmenge hydriert. Das Lösungsmittel wurde abdestilliert, der Rückstand chromatographisch gereinigt. Man erhielt 2,1 g öliges Produkt.

In analoger Weise wurden die Beispiele 7 und 8 der Tabelle 2 synthetisiert.

Tabelle 1 Allgemeine Formeln I und Ia mit X = $-\overset{\overset{O}{\|}}{C}-CH=CH-$

| Beispiel | $R^1$ | $R^2$ | 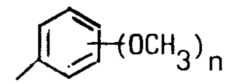 | allgemeine Formel | Fp. [°C] |
|---|---|---|---|---|---|
| 1 | $CH_3$ | $CH_3$ | 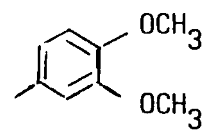 | 1 | 60 |
| 2 | $C_2H_5$ | $CH_3$ | 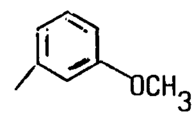 | I | Öl |
| 3 | $CH_3$ | $CH_3$ | " | I | Öl |
| 4 | $C_3H_7$ | $CH_3$ | 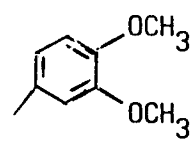 | I | Öl |
| 5 | $CH_3$ | $CH_3$ | " | Ia | 70 |

Tabelle 2 Allgemeine Formel I mit X = $-\overset{O}{\overset{\|}{C}}-CH_2-CH_2$

| Beispiel | $R^1$ | $R^2$ | 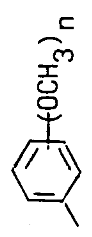 | Fp. [°C] |
|---|---|---|---|---|
| 6 | $C_2H_5$ | $CH_3$ | 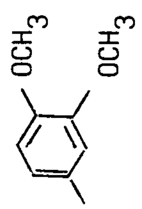 | Öl |
| 7 | $CH_3$ | $CH_3$ | 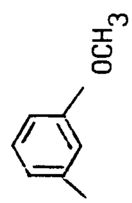 | Öl |
| 8 | $C_2H_5$ | $CH_3$ | " | Öl |

Beispiel 9

1-(4,7-Dimethoxy-6-[3-((2-(3,4-dimethoxyphenyl)ethyl)-methylamino)-propoxy]-benzofuranyl-5)-3-(4-hydroxyphenyl)propanol

11 g l-(4,7-Dimethoxy-6-/3-((2-(3,4-dimethoxyphenyl)ethyl)-methylamino)-propoxy/-benzofuranyl-5)-3-(-4-hydroxyphenyl)propenon wurden in 40 ml Ethanol / 7 g Pyridin bei RT mit 3,7 g $NaBH_4$ versetzt und 5 Stunden unter Rückfluß erhitzt. Die Mischung wurde auf Eiswasser gegossen, mit verd. HCl neutralisiert und mit $CH_2Cl_2$ extrahiert. Die organische Phase wurde getrocknet, das Lösungsmittel abdestilliert und der Rückstand an Kieselgel chromatographiert ($CH_2Cl_2$ : $CH_3OH$, 8 : 1).

Man erhielt 5,3 g Produkt, Öl.

**Patentansprüche**

**1.** Benzofuranderivate der Formeln I oder Ia

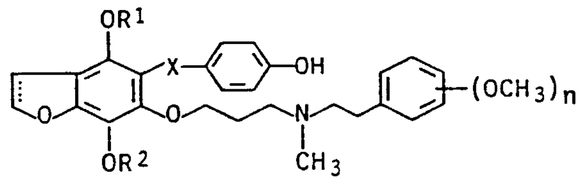

I

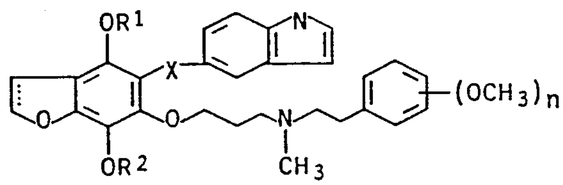

Ia

worin $R^1$ und $R^2$ unabhängig voneinander Wasserstoff, Alkyl oder Phenylalkyl mit jeweils 1 bis 4 C-Atomen im Alkylrest bedeuten,

X eine der Gruppen -CO-CH = CH-, -CO-$CH_2$-$CH_2$- oder -CHOH-$CH_2$-$CH_2$- darstellt,

n die Zahl 1 oder 2 ist und

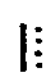 eine Einfach- oder Doppelbindung

darstellt.

2. Therapeutisches Mittel zur enteralen Anwendung, das neben üblichen galenischen Hilfsstoffen eine wirksame Menge eines Benzofuranderivates der Formel I oder Ia nach Anspruch 1 enthält.

3. Verwendung eines Benzofuranderivates der Formel I oder Ia nach Anspruch 1 zur Herstellung eines pharmazeutischen Mittels.

## Claims

1. A benzofuran derivative of the formula I or Ia

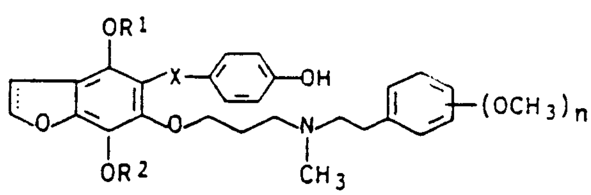

I

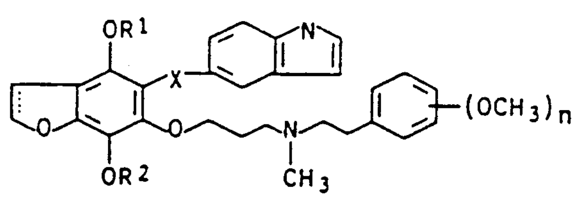

Ia

where $R^1$ and $R^2$ independently of one another are each hydrogen, alkyl or phenylalkyl where alkyl in each case is of 1 to 4 carbon atoms, X is -CO-CH = CH-, $-CO-CH_2-CH_2-$or $-CHOH-CH_2-CH_2-$,

n is 1 or 2 and 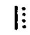

is a single or double bond.

2. A therapeutic agent for enteral use, which, in addition to conventional pharmaceutical auxiliaries, contains an effective amount of a benzofuran derivative of the formula I or Ia as claimed in claim 1.

3. Use of a bensofuran derivative of the formula I or Ia as claimed in claim 1 for the preparation of a pharmaceutical agent.

**Revendications**

1. Dérivés du benzofuranne, de formule I ou Ia

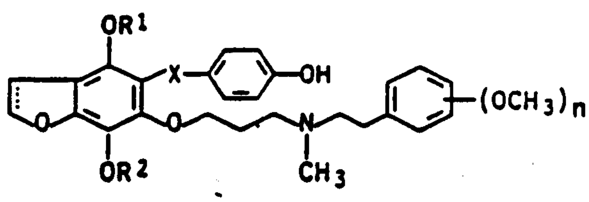

I

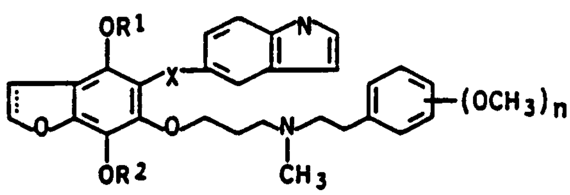

Ia

dans lesquelles $R^1$ et $R^2$ représentent chacun, indépendamment l'un de l'autre, l'hydrogène, un groupe alkyle en C 1-C 4 ou phényl-alkyle en C 1-C 4,

X représente l'un des groupes -CO-CH = CH-, $-CO-CH_2-CH_2-$ou $-CHOH-CH_2-CH_2-$,

n est égal à 1 ou 2, et

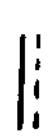 représente une liaison simple ou double.

2. Agent thérapeutique pour l'administration entérale, contenant, avec des produits auxiliaires galéniques usuels, une quantité efficace d'un dérivé du benzofuranne de formule I ou Ia selon la revendication 1.

3. Utilisation d'un dérivé du benzofuranne de formule I ou Ia selon la revendication I pour la préparation d'un produit pharmaceutique.